(19) Europäisches Patentamt
European Patent Office
Office européen des brevets

(11) Publication number: 0 036 436 A1

# (12) EUROPEAN PATENT APPLICATION

(21) Application number: **80101592.6**

(22) Date of filing: **26.03.80**

(51) Int. Cl.³: **C 07 C 177/00**, A 61 K 31/557

(43) Date of publication of application: **30.09.81**
**Bulletin 81/39**

(84) Designated Contracting States: **AT BE CH DE FR GB IT NL SE**

(71) Applicant: **MILES LABORATORIES INC., 1127 Myrtle Street, Elkhart Indiana 46514 (US)**

(72) Inventor: **Kluender, Harold Clinton, Dr., 17650 Tanager Lane, South Bend Indiana 46635 (US)**
Inventor: **Arndt, Henry Clifford, Dr., 23655 Cortland Drive, Elkhart Indiana 46514 (US)**

(74) Representative: **Dill, Erwin, Dr., c/o BAYER AG Zentralbereich Patente Marken und Lizenzen Bayerwerk, D-5090 Leverkusen 1 (DE)**

(54) **Methyl 11alpha,15R-dihydroxy-16,18-methano-16-methyl-9-oxoprost-13E-en-1-oate, a process for its production and composition containing it.**

(57) Disclosed is methyl 11α,15R-dihydroxy-16,18-methano-16-methyl-9-oxoprost-13E-en-1-oate of the formula:

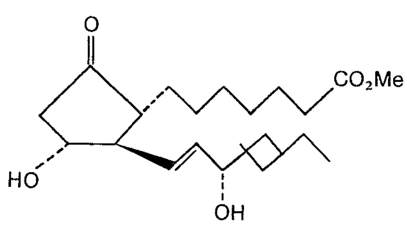

This compound is a potent bronchodilator and because it is less irritating to the tracheobronchical tract than $PGE_1$, it is useful in the treatment of asthma.

EP 0 036 436 A1

Miles Laboratories, Inc. Dn/Gh
Elkhart, Indiana/USA

**TITLE MODIFIED**
**see front page**

Methyl 11α, 15R-Dihydroxy-16,18-Methano-16-Methyl-9-Oxoprost-13E-En-1-Oate

## BACKGROUND OF THE INVENTION

### 1. Field of the Invention

The compound of this invention is an analog of a natural prostaglandin.

Natural prostaglandins are alicyclic compounds related to prostanoic acid, the structure of which is:

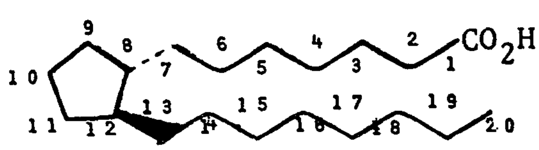

(I)

By convention, the carbon atoms of (I) are numbered sequentially from the carboxylic carbon atom. An important stereochemical feature of (I) is the *trans* orientation of the sidechains $C_1$-$C_7$ and $C_{13}$-$C_{20}$, an orientation common to all prostaglandins. In (I), as elsewhere in this specification, solid lines (——) provide a reference plane (such as the cyclopentyl ring or the bonds among $C_1$-$C_7$ and $C_{13}$-$C_{20}$); a dashed line (---) indicates projection of a covalent bond below such reference plane (*alpha*-configuration); while a wedged line (◢) represents direction above such plane (*beta*-configuration). These conventions apply to all structural formulae subsequently discussed in this specification. In some structures, however, a swung dash or serpentine line (∿) denotes orientation of a covalent bond either above or below the plane of reference (indicated by the Greek letter *xi* in the nomenclature of such structures).

Natural prostaglandins have the general structure,

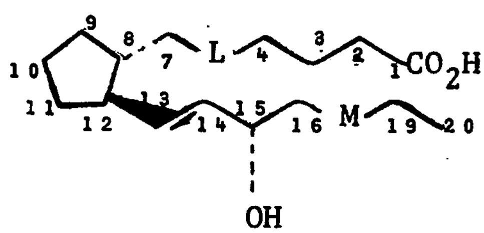

(II)

in which L and M may be ethylene or cis-vinylene radicals. Prostaglandins are characterized by the substituents on the cyclopentyl ring, the position of double bonds, if any, in the cyclopentyl ring and the number of double bonds in the side chains. When the cyclopentyl ring is fully saturated, carbonyl substituted at the 9-position and hydroxyl substituted at the 11-position an E-class prostaglandin is represented (PGE) and when there is a single double bond in the sidechains, i.e., L and M in Formula (II) are ethylene, a type-1 prostaglandin is represented. The naturally occurring E-class type-1 prostaglandin known as prostaglandin $E_1$ or $PGE_1$, is represented by the formula:

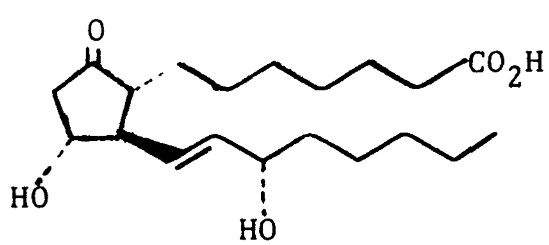

(III)

Recent research indicates that certain prostaglandins, including $PGE_1$ and analogs thereof, elicit biochemical and physiological effects in a variety of mammalian systems. In most mammalian respiratory tracts, PGE compounds affect *in vitro* preparations

of tracheal smooth muscle. The human lung normally contains PGE compounds. Consequently, some cases of bronchial asthma may involve an imbalance in the production or metabolism of these compounds.

## 2. Prior Art

Kurone et al., disclose in U.S. Patent 4,117,119 prostaglandins which are 15-cyclobutyl substituted. In Claim 1 of this patent, $PGE_1$ compounds which can be 1-methyl esters with a

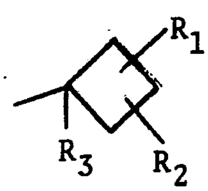

moiety attached to the 15-carbon atom where $R_1$ and $R_2$ represent hydrogen atoms and $R_3$ is alkyl of from 1 to 6 carbon atoms are disclosed. The compound claimed herein is closely related to those compounds disclosed by Kurone et al., but differs in that it contains an ethyl group in the 3-position of the cyclobutyl ring. Comparative tests between the 3-ethyl compound claimed herein and a compound of the type disclosed by Kurone

et al., in which the 15-cyclobutyl ring is 3-unsubstituted indicate that the present compound is clinically superior as a bronchodilator for use in treating asthma patients because it exhibits significantly less irritancy to the tracheobronchial tract.

## DETAILED DESCRIPTION

The preparation and comparative pharmacological testing of methyl 11α,15*R*-dihydroxy-16,18-methano -16-methyl-9-oxoprost-13*E*-en-1-oate (the compound claimed herein) and methyl 16, 19-cyclo-11α,15*R*-dihydroxy-16 -methyl-20-*nor*-9-oxoprost-13*E*-en-1-oate (the prior art compound which is closest in structure to this compound) are described in the following examples:

## EXAMPLE I

### Methyl 11α,15*R*-dihydroxy-16,18-methano-16-methyl -9-oxoprost-13*E*-en-1-oate (TR-4681)

A. Preparation of 2-Ethylpropane-1,3-diol

Lithium aluminum hydride (15.5 g, 0.4 mole) was slurried in 600 ml of dry ether. The slurry was cooled in an ice-water bath and then a solution of 37.6 g (0.2 mole) of diethyl ethylmalonate, obtained from the Aldrich

Chemical Company of Milwaukee, Wisconsin, in 100 ml of dry ether was added thereto. The mixture was heated to reflux for 3 hours and then cooled in an ice-water bath. Ethyl acetate (52 ml) was carefully added, followed by water (15.5 ml), which was followed by the addition of 15.5 ml of 15% aqueous sodium hydroxide solution and then 31 ml of water. This mixture was filtered and the solvents removed from the filtrate by evaporation *in vacuo* to yield 12.3 g of the title compound whose structure was confirmed by nuclear magnetic resonance, NMR.

B. Preparation of 2-Ethylpropane-1-3-di(*p*-toluenesulfonate)

A mixture of 12.3 g (0.118 mole) of 2-ethylpropane-1, 3-diol prepared in Step A and 350 ml of dry pyridine was prepared and cooled in an ice/water/salt bath. To this mixture was added 67.5 g (0.354 mole) of *p*-toluenesulfonyl chloride and the mixture was stirred for 3 hours at -15°C. The mixture was then poured into 3 liters of ice-cold 6N hydrochloric acid and the resultant mixture extracted with 1800 ml (3 x 600 ml) of ether. The ether extracts were combined and dried over anhydrous potassium carbonate-sodium sulfate, filtered and the solvent removed *in vacuo* to yield 48.1 g of the title compound the structure of which was confirmed by NMR.

C. Preparation of 3-Ethyl-1, 1-dicarbethoxycyclobutane

Sodium metal (7.6 g, 0.33 g/atom) was dispersed in 50 ml of dry xylene by heating to 130°C with rapid stirring. Dry xylene (168 ml) and 47.8 g (45.2 ml, 0.298 mole) of diethyl malonate were added and the mixture was allowed to react at 120°C. To the resultant mixture was added 48.1 g (0.116 mole) of 2-ethylpropane-1,3-di(*p*-toluenesulfonate) (prepared in Step B) dissloved in 120 ml of dry xylene.

This reaction mixture was heated to about 150°C and stirred for 18 hours whereupon the mixture was cooled and poured into 500 ml of water and extracted with 600 ml (2 x 300 ml) of ether. The aqueous material was made acidic with 10% aqueous HCl and then extracted with 600 ml (2 x 300 ml) of ether. The combined ether extract was washed with 200 ml of saturated aqueous sodium chloride, dried over anhydrous magnesium sulfate, filtered and the solvent removed *in vacuo*. Distillation of the product at reduced pressure yielded 7.0 g of the title compound whose structure was confirmed by NMR.

0036436

D. Preparation of 3-Ethylcyclobutane-1, 1-dicarboxylic acid

A solution of 7.0 g (0.031 mole) of 3-ethyl-1 -dicarbethoxycyclobutane (prepared in Step C) in 7 ml of absolute ethanol was prepared. To this solution there was added 6.87 g (0.123 mole) of potassium hydroxide dissolved in 76 ml of absolute ethanol and the mixture was heated to reflux with stirring for 1.5 hours. The mixture was then filtered and the filter cake washed with 10 ml of absolute ethanol and 75 ml of ether. The resulting filter cake was dissolved in 30 ml of ice-water and acidified with 36 ml of 50% aqueous sulfuric acid. The resulting mixture was cooled, filtered and dried to yield 4.5 g of the title compound whose structure was confirmed by NMR.

E. Preparation of 3-Ethylcyclobutanecarboxylic acid

The 3-ethylcyclobutane-1,1-dicarboxylic acid (prepared in Step D) (4.5 g, 0.026 mole) was decarboxylated by heating the compound to 190°C for about 15 minutes. Frothing accompanied this decarboxylation and when this ceased the mixture was cooled to yield approximately 3.3 g of the title compound whose structure was confirmed by NMR.

F. Preparation of 1-Methyl-3-ethyl-cyclobutanecarboxylic acid

A solution of 19.9 ml (140 mmol) of dry diisopropylamine in 100 ml of dry tetrahydrofuran was stirred under an argon atmosphere and 63.6 ml (140 mmol) of a solution of *n*-butyllithium (2.2 m) in hexane was added dropwise thereto while maintaining the temperature below about 5°C. The resultant mixture was then stirred for about 15 minutes with ice-bath cooling. A solution of 3.2 g (64 mmol) of 3-ethylcyclobutanecarboxylic acid (prepared in Step E) in 15 ml of dry tetrahydrofuran was added dropwise to the reaction mixture and stirred with cooling for 15 minutes. Methyl iodide (4.36 ml, 70 mmol) was added to the reaction mixture dropwise and the resultant mixture stirred without cooling for 2 hours. The mixture was then stirred with ice-methanol bath cooling as 10% hydrochloric acid was added dropwise until the resultant aqueous phase was acidic (about 25 ml was used). The aqueous phase was separated and extracted twice with ether. The combined ether extract was dried over anhydrous magnesium sulfate and the solvent removed *in vacuo* to yield 5 g of the title compound whose structure was confirmed by NMR.

G. Preparation of (1-methyl-3-ethylcyclobutyl) methyl ketone

A solution of 5.0 g (35 mmol) of 1-methyl-3-ethyl -cyclobutanecarboxylic acid (prepared in Step F) in 35 ml of dry ether was stirred with ice-bath cooling under an argon atmosphere and 40 ml of 2 M solution of methyllithium in ether was added thereto. The resultant mixture was stirred without cooling for 3 hours and then quenched by pouring the mixture into a vigorously stirred solution of 30 ml of methanol and 70 ml of water. The aqueous phase was separated and extracted twice with ether whereupon the combined ether extract was dried over anhydrous magnesium sulfate and evaporated. The residue was distilled at reduced pressure to yield 2.5 g of the title compound whose structure was confirmed by NMR.

H. Preparation of 1-Hydroxy 3-(3-ethyl-1-methyl-cyclobutyl)prop-1-en-3-one

Sodium hydride (1.25 g of 50% mineral oil dispension, 50 mmol) was washed with dry hexane and then stirred under an argon atmosphere with 1.25 ml of dry ether. A solution of 5 ml of methyl formite and 2.4 g of (1-methyl-3 -ethylcyclobutyl)methyl ketone (prepared in Step G) in 5 ml of ether was then added to the reaction mixture along with about 0.5 ml of methanol. As a voluminous

precipitate formed sufficient ether was added to make the reaction mixture more easily stirrable. The resultant mixture was stirred for 1 hour and then quenched by the addition of water. The other phase was separated and extracted three times with 1 M sodium hydroxide. The combined aqueous extraction phases were acidified with concentrated hydrochloric acid and extracted 3 times with ether. The combined ether extract was evaporated *in vacuo* and traces of water removed by the addition of benzene twice which was removed by evaporation *in vacuo* to provide the title compound whose structure was confirmed by NMR.

I. Preparation of 1-chloro-3-(3-ethyl -1-methylcyclobutyl)prop-1*E*-en-3-one

A solution of 5.8 g of 1-hydroxy-3-(3-ethyl-1 -methylcyclobutyl)prop-1-en-3-one (prepared in step H) in 100 ml of benzene was stirred under an argon atmosphere and 7 ml of thionyl chloride in 10 ml of benzene was added dropwise. The resulting solution was stirred for about 15 hours. Excess solvent was removed by distillation at atmospheric pressure and the residue was distilled *in vacuo* to yield 4.2 g of the title compound whose structure was confirmed by NMR.

J. Preparation of 1-iodo-3-(3-ethyl-1-methylcyclobutyl)prop-1*E*-en-3-one

A solution of 4.2 g of *trans*-1-chloro-3-(3-ethyl-1-methylcyclobutyl)prop-1*E*-en-3-one (prepared in Step I) and 10 g of anhydrous sodium iodide in 60 ml of acetone was refluxed with about 0.5 ml of $H_2SO_4$ for 4 hours under an argon atmosphere. The solvent was removed by evaporation *in vacuo* whereupon the residue was distilled with water and the aqueous mixture extracted several times with ether. The combined ether extract was washed with aqueous sodium thiosulfate solution and then dried over anhydrous magnesium sulfate and evaporated *in vacuo* to yield 6.8 g of the title compound whose structure was confirmed by NMR.

K. Preparation of 1-iodo-3-hydroxy-3-(3-ethyl-1-methylcyclobutyl)prop-1*E*-ene

A solution of 1-iodo-3-(3-ethyl-1-methylcyclobutyl)prop-1*E*-en-3-one (prepared in Step J) in absolute ethanol was prepared and cooled in a salt/ice/water bath. Sodium borohydride (1.51 g, 0.04 mole) was slurried in 50 ml of absolute ethanol and added to the cooled solution and the resulting mixture allowed to stir for 1 hour at 0°C.

The solvent was removed *in vacuo* and the residue taken up in 100 ml of water whereupon the aqueous mixture was extracted with 150 ml (3 x 50 ml) of ether. The combined ether extract was washed with 50 ml of saturated aqueous sodium chloride, dried over anhydrous magnesium sulfate, filtered and the solvent removed *in vacuo* to yield the title compound which was purified by chromatography on silica gel 60 using a chloroform elution to yield the title compound whose structure was confirmed by NMR.

L. The product of step K was blocked by converting the 3-hydroxy group to 3-(1-ethoxyethoxy) by reacting it with ethylvinylether and toluenesulfonic acid in dry ether followed by the addition of saturated aqueous sodium bicarbonate. Extraction with ether, separation, washing with saturated, aqueous sodium chloride, drying over anhydrous magnesium sulfate and evaporation *in vacuo* yielded 1-iodo-3-(1-ethoxyethoxy)-3-(3-ethyl-1-methyl-cyclobutyl)prop-1*E*-ene which was used in the next step.

M. Preparation of methyl 11α,15*R*-dihydroxy-16,18-methano-16-methyl-9-oxoprost-13*E*-en-1-oate

A first solution containing 0.9 g, 2.54 mmol of 1-iodo-3-(1-ethoxyethoxy)-3-(3-ethyl-1-methylcyclobutyl) prop-1*E*-ene in 9 ml of dry ether was prepared and cooled to -78°C. To this solution there was added 3.5 ml (5.08 mmol,...1.44 M) of *t*-butyllithium in pentane and the resulting mixture was stirred at -78°C. A second solution containing 0.332 g (2.54 mmol) of copper (I) pentyne in 50 ml of dry ether and 0.9 ml of dry hexamethylphosphorous triamide was prepared, stirred for 30 minutes and cooled to -78°C, whereupon the second solution was added to the first solution. A third solution containing 0.552 g (1.7 mmol) of methyl-7-[3α-(tetrahydropyran-2-yloxy)-5-oxocyclopent-1-enyl]heptanoate prepared as described in J. Amer. Chem. Soc., *95*:1676 (1973) in 9 ml of dry ether was prepared and added to the above mixture of the first and second solutions. The resulting mixture was stirred for 30 minutes at -78°C, for 90 minutes at -20°C and then quenched with 20% aqueous ammonium sulfate solution. This mixture was shaken for 10 minutes and the phases separated. The aqueous phase was extracted with 100 ml (2 x 50 ml) of ether. The combined organic phase was washed with cold 2% aqueous sulfuric acid and the wash layer was then back extracted with 100 ml (2 x 50 ml) of ether.

The organic extracts were combined and filtered through Celite (diatomaceous earth), washed with 50 ml of saturated aqueous sodium bicarbonate solution and then with 50 ml of saturated aqueous sodium chloride solution. The washed extract was dried over anhydrous magnesium sulfate and the solvent removed by evaporation *in vacuo*. The residue was stirred for 18 hours with 25 ml of acetic acid-water-tetrahydrofuran (65:35:10, V/V/V) at room temperature. The solvents were removed by evaporation *in vacuo* and the residue was taken up in 30 ml of water and 30 ml of ether-ethyl acetate. The aqueous material was extracted with 60 ml (2 x 30 ml) of ether-ethyl acetate whereupon the combined ether-ethyl acetate extract was washed with 30 ml of saturated aqueous sodium bicarbonate and then with 30 ml of saturated aqueous sodium chloride. The extract was dried over anhydrous magnesium sulfate and the solvent was removed by evaporation *in vacuo* to yield 0.6482 g of a product having the structure:

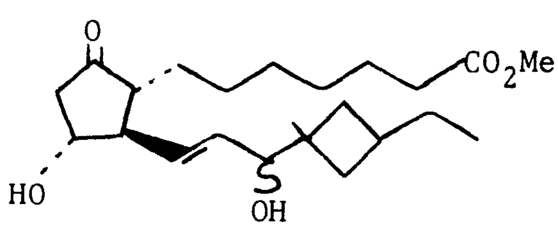

which is a mixture of the 15*R* and *S* hydroxy isomers. The isomeric mixture was separated by chromatography which resulted in 72.8 mg of the more polar isomer as a yellow oil which was confirmed as being the title compound by nuclear magnetic resonance, infrared and mass spectral analysis.

## EXAMPLE II

### Methyl 16,19-cyclo-11α,15*R*-dihydroxy-16-methyl -20-*nor*-9-oxoprost-13*E*-en-1-oate (TR-4690)

A. Preparation of 1-methylcyclobutanecarboxylic acid

This intermediate was prepared as in Step F of Example I by replacing 3-ethylcyclobutanecarboxylic acid with cyclobutanecarboxylic acid obtained from the Aldrich Chemical Company of Milwaukee, Wisconsin to yield the product whose structure was confirmed by NMR.

B. Preparation of (1-methylcyclobutyl)methyl ketone

The title compound was prepared according to the procedure of Step G (Example I) by replacing 1-methyl -3-ethylcyclobutanecarboxylic acid with 1-methylcyclobutanecarboxylic acid (prepared in Step II A) to yield a product whose structure was confirmed by NMR.

C. Preparation of 1-hydroxy-3-(1-methylcyclobutyl) -prop-1-en-3-one

The title compound was prepared according to the procedure set out in Step H (Example I) except that (1 -methylcyclobutyl)methyl ketone prepared in Step II B was used in place of (1-methyl-3-ethylcyclobutyl)methyl ketone to provide a product whose structure was confirmed by NMR.

D. Preparation of 1-chloro-3-(1-methyl-cyclobutyl)prop-1*E*-en-3-one

The title compound was prepared according to the procedure of Step I (Example I) by replacing 1-hydroxy -3-(3-ethyl-1-methylcyclobutyl)prop-1-en-3-one with 1-hydroxy-3-(1-methylcyclobutyl)prop-1-en-3-one prepared in Step II C to provide a product whose structure was confirmed by NMR.

E. Preparation of 1-iodo-3-(1-methylcyclobutyl) -prop-1*E*-en-3-one

The title compound was prepared according to the procedure of Step J (Example I) by replacing 1-chloro-3 -(1-methylcyclobutyl)prop-1*E*-en-3-one with 1-chloro-3 -(1-methylcyclobutyl)prop-1*E*-en-3-one prepared in Step II D to provide a product whose structure was confirmed by NMR.

F. Preparation of 1-iodo-3-hydroxy-3-(1-methylcyclobutyl)prop-1*E*-ene

The title compound was prepared according to the procedure of Step K (Example I) by replacing 1-iodo-3-(3-ethyl-1-methylcyclobutyl)prop-1*E*-en-3-one with 1-iodo-3-(1-methylcyclobutyl)prop-1*E*-en-3-one prepared in Step II E to provide a product whose structure was confirmed by NMR.

G. The product of Step F was blocked by converting the 3-hydroxy group to 3-(1-ethoxyethoxy) by reacting it with ethylvinylether and toluensulfonic acid in dry ether followed by the addition of saturated aqueous sodium bicarbonate. Extraction with ether, separation, washing with saturated, aqueous sodium chloride, drying over anhydrous magnesium sulfate and evaporation *in vacuo* yielded 1-iodo-3-(1-ethoxyethoxy)-3-(1-methylcyclobutyl) prop-1*E*-ene.

H. Preparation of methyl 16,19-cyclo-11α,15*R*-dihydroxy-16-methyl-20-*nor*-9-oxoprost-13*E*-en-1-oate

The synthesis described in Step M (Example I) was repeated in a similar manner except that 1-iodo-3-(1-ethoxyethoxy)-3-(3-ethyl-1-methylcyclobutyl)prop-1*E*-ene was replaced with 1-iodo-3-(1-ethoxyethoxy)-3-(1-methylcyclobutyl)prop-1*E*-ene to provide 1.0548 g of a mixture of isomers of the formula:

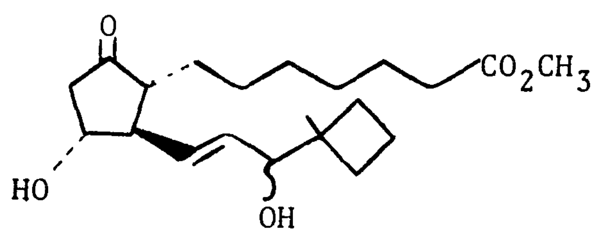

The isomeric mixture was separated by chromatography which resulted in 127.0 mg of the more polar isomer as a yellow oil which was confirmed as being the title compound by nuclear magnetic resonance, infrared and mass spectral analysis.

DETERMINATION OF PHARMACOLOGICAL ACTIVITY

The compounds prepared as described in Examples I and II, i.e., TR-4681 and TR-4690, were tested to determine their activity in the relaxation of the human bronchial muscle relative to $PGE_1$. The results of this test indicated a relative potency of TR-4681 to $PGE_1$ of 1.33 and a relative potency of TR-4690 of 3.9 indicating that TR-4690 is approximately 3 times more potent than TR-4681 as a bronchodilator. If potency as a bronchodilator were the only criterion upon which a drug of this type were judged, TR-4690 would be preferred. However, since $PGE_1$ was found to elicit sufficient cough and sore throat to preclude its clinical use, a second screen has been developed to determine the irritancy of prostaglandins to the tracheobronchial tract. This screen and the results observed in using it to test TR-4681 and TR-4690 are as follows:

MATERIALS

$PGE_1$ and the test compounds were dissolved in absolute ethanol to give stock solutions of 10 mg/ml. These stock solutions and an ethanol vehicle were diluted using isotonic saline adjusted with phosphate buffer to pH 7.2.

METHOD

Specific pathogen free cats (Category III) of either sex from the Laboratory Animals Centre were used. Conscious cats (2-4 kg) were restrained in an adjustable Perspex box with their heads protruding into a separate Perspex chamber with a seal around the neck. The respiratory pattern and rate of each cat was recorded via a Fleisch head pneumotachograph protruding into the top of the chamber. Sound was recorded by a microphone also protruding into the chamber. An aerosol of 1-8μm particle size was generated by a Monaghan 670 Ultrasonic nebulizer and passed to the cat's face via an opening in the front of the chamber. Each cat was challenged initially for 10 minutes with an aerosol of isotonic saline adjusted with phosphate buffer to pH 7.2 plus the appropriate concentration of ethanol. This control challenge was followed by a 10 minute aerosol challenge of the test compound of $PGE_1$ in the appropriate vehicle. This 10 minute challenge period is much greater than the brief period of inhalation likely to be used in man, but it does produce a larger number of coughs and enables us to determine the potency of the analogue relative to $PGE_1$ in producing other unwanted side effects.

Seven days were left between successive challenges to avoid the development of tolerance. Both drugs were tested in 5 cats using a wide range of concentrations of aerosol stock solutions. Each cat was observed during and up to 24 hours after the experiment for other side effects, e.g., sedation, diarrhea, and salivation.

The results of these tests are set out in Tables I and II:

TABLE I

SUMMARY OF TR-4681 RESULTS

| Conc (μg/ml) | $PGE_1$ %ΔRR | Cough | TR-4681 %Δ RR | Cough |
|---|---|---|---|---|
| 1.0 | + 9 | 2 | 0 | 0 |
| 10.0 | +34 | 3 | +3 | 0 |
| 50.0 | +45 | 69 | +41 | 16 |
| 100.0 | +38 | 33 | +41 | 11 |
| 500.0 | +47 | 58 | +67 | 14 |

% ΔRR = percent change in respiratory rate

TABLE II

SUMMARY OF TR-4690 RESULTS

| Conc (μg/ml) | $PGE_1$ % ΔRR | Cough | TR-4690 % ΔRR | Cough |
|---|---|---|---|---|
| 1.0 | +7 | 7 | +5 | 6 |
| 10.0 | +20 | 10 | +11 | 5 |
| 50.0 | +10 | 11 | +26 | 15 |
| 100.0 | +31 | 31 | +21 | 1 |
| 500.0 | +42 | 63 | +39 | 33 |

% ΔRR = percent change in respiratory rate

The data appearing in Tables I and II was compared with a computer program of relative potencies using a multiple assay of several test preparations against a standard and from this comparison it was determined that TR-4681 is approximately 50 times less potent an irritant than $PGE_1$ and that TR-4690 is approximately 2 times less potent an irritant than $PGE_1$ thus rendering TR-4681 twenty-five times less potent an irritant to the tracheobronchial tract than TR-4690.

From the results of the foregoing tests, one would unequivocally choose TR-4681 over TR-4690 for treatment of asthma patients because, while TR-4690 is approximately 3 times more potent than TR-4681 in relaxing human bronchial muscle, it is approximately 25 times more potent an irritant, which side effect would be highly adverse to the clinical use of TR-4690 in the treatment of asthma.

CLAIMS:

1. Methyl 11α,15R-dihydroxy-16,18-methano-16-methyl-9-oxoprost-13E-en-1-oate.

2. Composition containing the compound of claim 1.

3. A process for the manufacture of methyl 11α,15R-dihydroxy-16,18-methano-16-methyl-9-oxoprost-13E-en-1-oate which comprises

a) preparing 2-ethylpropane-1,3-diol;

b) converting the compound of a) into 2-ethylpropane-1-3-di(p-toluenesulfonate);

c) converting the compound of b) into 3-ethyl-1,1-dicarbethoxycyclobutane;

d) converting the compound of c) into 3-ethylcyclobutane-1,1-dicarboxylic acid;

e) converting the compound of d) into 3-ethylcyclobutanecarboxylic acid;

f) converting the compound of e) into 1-methyl-3-ethyl-cyclobutanecarboxylic acid;

g) converting the compound of f) into (1-methyl-3-ethylcyclobutyl)methyl ketone;

h) converting the compound of g) into 1-hydroxy 3-(3-ethyl-1-methyl-cyclobutyl)prop-1-en-3-one;

i) converting the compound of h) into 1-chloro-3-(3-ethyl-1-methylcyclobutyl)prop-1E-en-3-one;

j) converting the compound of i) into 1-iodo-3-(3-ethyl-1-methylcyclobutyl)prop-1E-en-3-one;

k) converting the compound of j) into 1-iodo-3-hydroy-3-(3-ethyl-1-methylcyclobutyl)-prop-1E-ene;

l) converting the 3-hydroxy group of the compound of k) into a 3-(1-ethoxyethoxy) group; and

m) converting the compound of l) into the desired compound.

European Patent Office

# EUROPEAN SEARCH REPORT

0036436

Application number

EP 80 10 1592

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| DX | US - A - 4 117 119 (ONO PHARMACEUTICAL)<br>* Claims *<br>-- | 1,2 | C 07 C 177/00<br>A 61 K 31/557 |
| X | PROSTAGLANDINS, vol. 16, no. 1 (07-1978) pages67-77<br>H.C. ARNDT et al.: "The synthesis and biological activity of ω-pentanor-15-alkylcyclobutyl-PGE$_1$ analogs"<br>* Pages 67-71; table 1, page 71, structure 3g *<br>---- | 1-3 | |

TECHNICAL FIELDS SEARCHED (Int. Cl.3)

C 07 C 177/00
A 61 K 31/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 30-10-1980 | BERTE |